Europäisches Patentamt
European Patent Office
Office européen des brevets

(19)

(11) Publication number: **0 414 903 A1**

(12) **EUROPEAN PATENT APPLICATION published in accordance with Art. 158(3) EPC**

(21) Application number: **90900987.0**

(22) Date of filing: **14.12.89**

(86) International application number: **PCT/JP89/01256**

(87) International publication number: **WO 90/06919 (28.06.90 90/15)**

(51) Int. Cl.[5]: **C07D 277/06**, C07D 417/14, C12N 9/99, A61K 31/425

(30) Priority: **14.12.88 JP 313837/88**

(43) Date of publication of application: **06.03.91 Bulletin 91/10**

(84) Designated Contracting States: **AT BE CH DE ES FR GB IT LI LU NL SE**

(71) Applicant: **KABUSHIKI KAISHA YAKULT HONSHA**
**1-19, Higashishinbashi 1-chome**
**Minato-ku Tokyo 105(JP)**

(72) Inventor: **SAWADA, Seigo**
**K.K. Yakult Honsha 1-19, Higashishinbashi 1-chome**
**Minato-ku Tokyo 105(JP)**
Inventor: **OKAJIMA, Satoru**
**K.K. Yakult Honsha 1-19, Higashishinbashi 1-chome**
**Minato-ku Tokyo 105(JP)**

(74) Representative: **Vossius & Partner**
**Siebertstrasse 4 P.O. Box 86 07 67**
**W-8000 München 86(DE)**

(54) **NEW THIOPROLINE DERIVATIVE.**

(57) New thioproline derivatives of the general formula

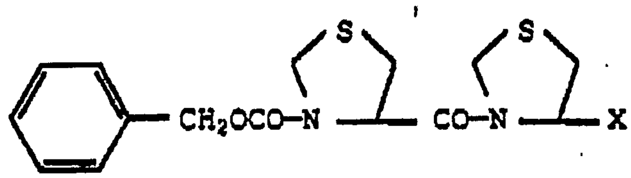

(I)

wherein X stands for an dialkyl acetal group or other different groups derived from the CHO group, and an anti-amnestic agent and a proline endopeptidase inhibitor containing these compounds as active ingredient.

EP 0 414 903 A1

# NEW THIOPROLINE DERIVATIVES

## Technical Field

The present invention relates to new proline derivatives useful as medicaments or intermediates therefor.

## Background Art

Tsuru, Yoshimoto et al. have recently revealed that those substances having proline skeleton at their N-terminal, e.g. N-benzyloxycarbonyl-L-prolyl-L-prolinal, have inhibitory activity to proline endopeptidase as well as an anti-amnestic action (Japanese Laid-open Patent Appln. No. Sho. 60-188317). These substances are expected by many to open a way to the treatment of senile dementia, for example Arzheimer's dementia which is now increasing, and attempts of synthesis have been continuing to seek more potent proline endopeptidase inhibitors. (Japanese Patent Appln. Sho. 62-225377 and 62-327181.) Among these groups of compounds, benzyloxy carbonyl thioprolinal (see formula (II) below) has been found to possess outstanding such activity.

These aldehyde group-containing derivatives, however, are chemically unstable in nature due to the aldehyde group. There is therefore a demand to develop compounds of higher chemical stability.

## Disclosure of Invention

As a result of extensive researches, we have succeeded, by converting benzyloxycarbonylthioprolyl-thioprolinal of the formula (II)

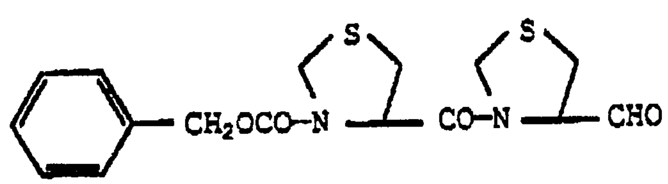

(II)

into different aldehyde-protected derivatives, in obtaining compounds which are more stable than in the free aldehyde form, while retaining a strong inhibitary action on proline endopeptidase.

Accordingly, the present invention provides new thioproline derivatives of the general formula (I)

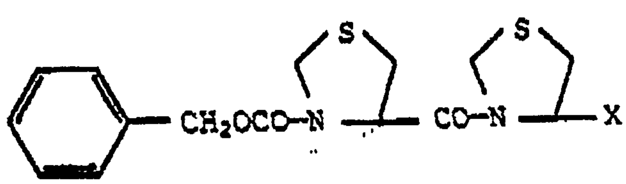

(I)

wherein X is a dialkyl acetal group, a dialkyl thioacetal group, a cyclic acetal group, a cyclic dithioacetal group, a cyclic thioaminal group or a diacyloxymethyl group; a hydroxyiminomethyl group, a lower-alkylaminoiminomethyl group, an arylaminoiminomethyl group or a carbamoylaminoiminomethyl group; or a 2-benzimidazole group.

Furthermore, the present invention provided an anti-amnestic agent comprising at least one thioproline derivatives of the general formula (I) shown above as active ingredient thereof.

Still furthermore, the present invention provides a proline endopeptidase inhibitor comprising at least one thioproline derivatives of the general formula (I) shown above as active ingredient thereof.

The new thioproline derivatives of the general formula (I) according to the present invention may be synthesized by subjecting the aldehyde of the formula (II) shown above as the starting material to a customary protective group-introducing reaction applicable to aldehydes.

Thus, for example, the above-mentioned aldehyde may be reacted in the presence of an appropriate acid catalyst with alcohols, thiols, diols, dithiols or aminothiols to give corresponding dialkyl acetals, dialkyl thioacetals, cyclic acetals, cyclic dithioacetals or thioaminals. In carrying out this, an appropriate solvent may be used where necessary.

The above-mentioned aldehyde may also be reacted with acylation reagents such as acid anhydrides or acid halides to give corresponding diacyl acetals.

The above-mentioned aldehyde may also be reacted with hydroxylamine, hydrazine, alkylhydrazines or arylhydrazines to give corresponding oxime, hydrazone, alkylhydrazones or arylhydrazones. In carrying out this, an appropriate acid or alkali catalyst or a reaction solvent may be used where necessary.

The above-mentioned aldehyde may also be reacted with semicarbazide, thiosemicarbazide or the like to give corresponding semicarbazone, thiosemicarbazone or the like.

The above-mentioned aldehyde may also be converted into the 2-benzimidazole derivative through reaction with ortho-phenylenediamine to form the imidazole ring.

The new compounds of the present invention as well as their preparation will now be illustrated in more detail below by way of examples.

The benzyloxycarbonylthioprolylthioprolinal used in these examples can be prepared by reacting N-benzyloxycarbonyl-L-thioproline with L-thioprolinol and then oxidizing the resultant alcohol (of the formula I wherein X stands for group-$CH_2OH$) to convert the group-$CH_2OH$ into group-CHO (see Preparative Example shown below).

Example 1: Benzyloxycarbonyl-L-thioprolyl-L-thioprolinal dimethyl acetal

Benzyloxycarbonyl-L-thioprolyl-L-thioprolinal (hereinafter referred to as ZTT) (1.0 g, 2.73 mmol) is dissolved in methanol (20 ml), and a HCl-saturated methanolic solution (5 ml) is added at room temperature. The mixture is stirred for 15 minutes and then heated under reflux for 30 minutes. It is then allowed to cool and the solvent is distilled off under reduced pressure. The residue is purified by column chromatography on silica gel (20% ethyl acetate/benzene), whereby ZTT dimethyl acetal is obtained as a colorless syrup (450 mg ; yield 40.0%).

MS(m/e) : 412($M^+$).

IR ($CHCl_3$) : 2990, 2930, 1645, 1410, 1350, 1110, 1075.

NMR(in $CDCl_3$) : 3.15 - 3.55 and 4.10 - 5.20(19H, m), 7.26 - 7.46(5H, m).

Example 2: ZTT diethyl acetal

ZTT(1.0 g, 2.73 mmol) is dissolved in ethanol (20 ml) and a HCl-ethanol solution (5 ml) is added. The mixture is stirred for 15 minutes and then heated under reflux for 30 minutes. It is then allowed to cool and worked up as in Example 1 to give ZTT diethyl acetal as a colorless syrup (310 mg; yield 25.8%).

MS(m/e) : 440($M^+$).

IR ($CHCl_3$): 2970, 1695, 1640, 1405, 1350, 1100, 1055.

NMR(in $CDCl_3$) : 1.19 - 1.57(6H, m), 3.06 - 3.85 and 4.10 - 5.19(17H, m), 7.28 - 7.40(5H, m)

Example 3: ZTT dibutyl acetal

ZTT (1.0 g, 2.73 mmol) is dissolved in n-butanol (10 ml) and $BF_3 \cdot Et_2O$(0.84 ml) is added dropwise at room temperature. The mixture is stirred for 2 hours and the solvent is distilled off under reduced pressure. The residue is purified by column chromatography on silica gel (20% ether/n-hexane), whereby ZTT dibutyl acetal is obtained as a colorless syrup (690 mg ; yield 50.9%).

MS(m/e) : 496($M^+$).

IR($CHCl_3$) : 2950, 2850, 2240, 1695, 1645, 1405, 1350, 1105, 900, 700, 645.

NMR (in $CDCl_3$) : 0.89 - 0.97(6H, m), 1.25 - 1.46(4H, m), 1.46 - 1.64(4H, m), 3.00 - 3.80 and 4.10 - 5.25-(17H, m), 7.28 - 7.40(5H, m).

Example 4: ZTT dimethylenedioxy acetal

ZTT (1.0 g, 2.73 mmol) is dissolved in THF(20 ml) and ethylene glycol(178 mg, 2.87 mmol) is added. $BF_3 \cdot Et_2O$(0.84 ml) is added dropwise and the mixture is stirred at room temperature for 7 hours. The solvent is distilled off under reduced pressure. The residue is worked up as in Example 3 to obtain the title compound as a colorless syrup (680 mg ; yield 60.7%).

MS(m/e) : 410($M^+$).
IR($CHCl_3$) : 2985, 2875, 1700, 1645, 1410, 1350, 1175, 1110.
NMR(in $CDCl_3$) : 2.90 - 5.25(17H, m), 7.27 - 7.40 (5H, m).

Example 5: ZTT trimethylenedioxy acetal

ZTT (1.0 g, 2.73 mmol) and 1,3-propanediol (218 mg, 2 87 mmol) are used to effect reaction and after-treatment as in Example 4, whereby the title compound is obtained as a colorless syrup (1.10 g ; yield 95.0%).

MS(m/e) : 424($M^+$).
IR ($CHCl_3$) : 2980, 2950, 1695, 1410, 1350, 1140, 1110.
NMR(in $CDCl_3$):3.10 - 5.30(19H, m), 7.26 - 7.42(5H, m).

Example 6: ZTT dimethylene dithioacetal

ZTT (1.0 g, 2.73 mmol) is dissolved in methylene chloride (20 ml) and 1,2-ethanedithiol (257 mg, 2.73 mmol) is added. $BF_3 \cdot Et_2O$ (0.84 ml) is added dropwise and the mixture is stirred at room temperature for 1 hour. The solvent in the mixture was distilled off under reduced pressure and the mixture is purified by column chromatography on silica gel (50% ether/n-hexane), whereby the title compound is obtained as a colorless syrup (290 mg ; yield 24.0%).

MS(m/e) : 442($M^+$).
IR ($CHCl_3$) : 2240, 1695, 1645, 1405, 1350, 1090, 900, 700, 650.
NMR(in $CDCl_3$) : 3.05 - 5.30(17H, m), 7.26 - 7.44(5H, m).

Example 7; ZTT trimethylene dithioacetal

ZTT (1.0 g, 2.73 mmol) is dissolved in methylene chloride (20 ml) and 1,3-propanedithiol (325 mg, 3.01 mmol) is added. $BF_3 \cdot Et_2O$ (0.84 ml) is added dropwise and the mixture is stirred at room temperature For 1 hour. The solvent in the mixture is distilled off under reduced pressure and the residue is purified by column chromatography on silica gel (10% ethyl acetate/benzene), whereby the title compound is obtained (520 mg ; yield 41.7%).

MS(m/e) : 456($M^+$).
IR ($CHCl_3$) : 2980, 2930, 2890, 1700, 1650, 1410, 1350, 1175.
NMR(in $CDCl_3$) : 2.40 - 5.40(19H, m), 7.27 - 7.42(5H, m, Ph-H).

Example 8: ZTT dimethylene thioaminal

ZTT (1.0 g, 2.73 mmol) is dissolved in methanol (20 ml) and aminoethanethiol (218 mg, 2.87 mmol) is added. One drop of concentrated sulfuric acid is added and the mixture is stirred at room temperature for 5 hours. The solvent in the reaction mixture is distilled off under reduced pressure and the residue is purified by column chromatography on silica gel (10% ethyl acetate/benzene) to give the title compound (590 mg ; yield 50.8%).

MS(m/e) : 425($M^+$).
IR ($CHCl_3$) : 2975, 2920, 2870, 1695, 1660, 1405, 1350, 1170.
NMR(in $CDCl_3$) : 2.79 - 3.58 and 4.20 - 5.22(18H, m), 7.26 - 7.42(5H, m, Ph-H).

Example 9: ZTT aldoxime

ZTT (1.0 g, 2.73 mmol) is dissolved in ethanol (15 ml), and hydroxylamine hydrochloride (209 mg, 3.01

mmol) and pyridine (5 ml) are added. The mixture is stirred at room temperature for 12 hours. The solvent in the reaction mixture is distilled off under reduced pressure and the residue is purified by column chromatography on silica gel (50% ether/n-hexane) to obtain the title compound (950 mg ; yield 91.3 %).

MS(m/e) : 381($M^+$).

IR ($CHCl_3$) : 3545, 3300, 2990, 2930, 1690, 1655, 1410, 1350, 1160, 1100, 955, 910, 690.

NMR(in $CDCl_3$) : 2.80 - 3.55 and 4.25 - 5.60(14H, m), 7.26 - 7.44(5H, m, Ph-H).

Example 10; ZTT semicarbazone

ZTT(1.0 g, 2.73 mmol) is dissolved in methanol (20 ml), and water (3 ml), semicarbazide hydrochloride (351 mg, 3.00 mmol) and sodium acetate (246 mg) are added. The mixture is stirred at room temperature for 2 hours. The solvent in the reaction mixture is distilled off under reduced pressure and the residue is purified by column chromatography on silica gel (3% methanol/chloroform) to obtain ZTT semicarbazone (660 mg ; yield 57.1%).

MS(m/e) : 423($M^+$).

IR ($CHCl_3$) : 3500, 3390, 3340, 2985, 2920, 1690, 1565, 1405, 1350, 1175, 1105.

NMR(in $CDCl_3$) : 3.00 - 3.70 and 4.45 - 5.25(16H, m), 7.20 - 7.45(5H, m, Ph-H).

Example 11: ZTT phenylhydrazone

ZTT (1.0 9, 2.73 mmol) is dissolved in ethanol (15 ml), and water (2 ml), phenylhydrazine hydrochloride (324 mg, 3.00 mmol) and sodium acetate (246 mg) are added. The mixture is stirred at room temperature for 30 minutes. After the reaction is complete, the solvent is distilled off under reduced pressure, and the residue is purified by column chromatography on silica gel (30% ethyl acetate/benzene) to obtain ZTT phenylhydrazone (490 mg ; yield 39.3%).

MS(m/e) : 456($M^+$).

IR ($CHCl_3$) : 2985, 2920, 1695, 1600, 1490, 1405, 1350, 1250, 1175, 1100.

NMR(in $CDCl_3$) : 2.95 - 3.60 and 4.35 - 5.30(14H, m), 6.80 - 7.10(5H, m), 7.20 - 7.43(5H, m).

Example 12: ZTT diacetylacetal

ZTT (1.0 g, 2.73 mmol) is dissolved in acetic anhydride (10 ml), and $BF_3 \cdot Et_2O$ (0.84 ml) is added. The mixture is stirred at room temperature for 2 hours. The solvent in the reaction mixture is distilled off under reduced pressure and the residue is purified by column chromatography on silica gel (20% ethyl acetate/benzene) to give ZTT diacetyl acetal (280 mg ; yield 21.9%).

MS(m/e) : 468($M^+$).

IR ($CHCl_3$) : 2990, 2930, 1765, 1695, 1405, 1340, 1205, 1080, 1010.

NMR(in $CDCl_3$) : 2.09 and 2.10(3H, s, and 3H, s, OAcX2). 3.00 - 3.45 and 4.30 - 5.25(13H, m), 7.25 - 7.40(5H, m).

Example 13: N-(Benzyloxycarbonylthioprolyl)-4-(2-benzimidazoyl)-thiazolidine

ZTT (1.0 g, 2.73 mmol) is dissolved in methanol (20 ml), and o-phenylenediamine (325 mg, 3.01 mmol) and 50% acetic acid (five drops) are added. The mixture is stirred at room temperature for 12 hours. The solvent in the reaction mixture is distilled off under reduced pressure and the residue is purified by column chromatography on silica gel (2% methanol/chloroform) to give the title compound (see the formula (III) below) (260 mg ; yield 20.9%).

MS(m/e) : 454($M^+$).

IR ($CHCl_3$) : 3210, 2980, 2930, 1670, 1410, 1350, 1270, 1180.

NMR(in $CDCl_3$) ; 3.33(2H,d), 3.51(1H,dd),3.87(1H,d), 4.62 and 4.78(2H,d), 4.72 and 4.95(2H,d), 4.85(1H,t), 5.23 and 5.35(2H, d,J=12.1HZ), 6.07(1H,d), 7.16 - 7.43(9H,m).

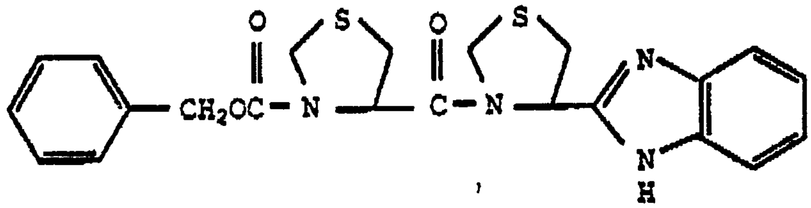 (III)

Preparative Example 1

Preparation of N-benzyloxycarbonyl-L-thioprolyl-L-thioprolinol

(1) Preparation of N-benzyloxycarbonyl-L-thioproline

L-Thioproline (25 g) is dissolved in 2N NaOH (94 ml), aid ether (20 ml) is added under ice-cooling and stirring. To the solution are simultaneously added dropwise benzyloxycarbonyl chloride (38 ml) and 4N NaOH (70 ml) over a period of 30 minutes with stirring. The mixture is stirred at room temperature for 2 hours, washed with ether (100 ml) and adjusted with 6N HCl to pH2. It is extracted with ethyl acetate (200 ml and 100 ml) and the ethyl acetate layer is washed with water (50 ml), dried over $MgSO_4$ and filtrated. The filtrate is concentrated to dryness under reduced pressure to obtain an oily substance. Yield: 51 g (100%)

(2) Preparation of N-benzyloxycarbonyl-L-thioprolyl-L-thioprolinol

The oily substance obtained in (1) (29 g), triethylamine (45 ml) and L-thioprolinol (12.9 g) are dissolved in methylene chloride (700 ml) at room temperature with stirring. To the solution is added dropwise a solution of DMC (30 9) in methylene chloride (330 ml) over a period of one hour. The mixture is stirred at room temperature for one hour and then washed successively with water, a $NaHCO_3$-saturated aqueous solution, IN HCl and water, and the methylene chloride layer is dried over $MgSO_4$ and filtrated. The filtrate is concentrated to dryness under reduced pressure and the residue is crystallized from ether to obtain the title compound.

mp : 127°C, $[\alpha]_D^{25°C}$ = -129.77 ($CHCl_3$)

Yield : 34 g (85.2%)

Preparative Example 2

Preparation of N-benzyloxycarbonyl-L-thioprolyl-L-thioprolinal

Under an atmosphere of nitrogen, phosgene (7.5 ml) is added to methylene chloride (150 ml) at -60°C with stirring. To the mixture is added dropwise a solution of DMSO (6.2 ml) in methylene chloride (150 ml) with stirring over a period of 60 minutes. After stirring for a further 5 minutes, a solution of N-benzyloxycarbonyl-L-thioprolyl-L-thioprolinol (16 g) prepared in Preparative Example 1 in methylene chloride (200 ml) is added dropwise over a period of 40 minutes, and the mixture is stirred for a further 30 minutes. Triethyl-amine (55 ml) is added dropwise and the mixture is stirred until it reaches room temperature. The mixture is washed twice with water, dried over $MgSO_4$ and filtrated. The filtrate is concentrated to dryness under reduced pressure and the residue is subjected to column chromatography on silica gel with the solvent chloroform : methanol = 50 : 1 as eluant. The eluate is concentrated to dryness under reduced pressure. To the residue are added ethanol (41 ml), sodium hydrogen sulfate (48 g) and water (84 ml), and the mixture is stirred for 15 minutes. The ethanol is then distilled off and the residue

is washed with ether. The aqueous layer is adjusted to pH9 with an aqueous solution of potassium carbonate, extracted with chloroform (200 ml), dried over $MgSO_4$ and filtrated. The filtrate is concentrated to dryness under reduced pressure to give the title substance as an oil (semi-solid).

$$[\alpha]_D^{20°C} = -102.20 (CHCl_3)$$

Yield : 6.1 g(38.3%)

The novel thioproline derivatives of the general formula (I) shown above have an excellent inhibitory action on proline endopeptidase.

Accordingly, the present invention provides a proline endopeptidase inhibitor comprising at least one thioproline derivative of the general formula (I) shown above as active ingredient thereof.

It has also been found that the thioproline derivatives of the general formula (I) possess an anti-amnestic action.

Accordingly, the present invention also provides an anti-amnestic agent comprising at least one thioproline derivative of the general formula (I) shown above as active ingredient thereof.

The above-mentioned inhibitory activity against proline endopeptidase was demonstrated in the manner as described below.

The thioproline derivatives of the general formula (I) shown above as test compound were assayed for inhibitory activity against bovine brain PEP by the following test procedure using as substrate N-benzyloxycarbonyl-glycyl-proline-β-naphthylamide (Z-Gly-Pro-β-NA).

Assay method

100 µl of PEP (about 0.14 U/ml) and 100µl of one of test compound solutions adjusted to different concentrations (0, $10^{-9}$ - $10^{-4}$M) were added to 0.7 ml of 20 mM Tris-HCl buffer (pH = 7.0) containing 10 mM EDTA and 10 mM 2-mercaptoethanol, and the mixture was preincubated at 37° C for 5 minutes. To the mixture is added 100 µl of one of substrate solutions in 40% dioxane at different concentrations (5.0, 2.5, 1.25, 0.625, 0.3125 mM) and the resultant mixture was incubated again at 37° C for 15 minutes to allow the enzymatic reaction to proceed. The reaction was allowed to proceed using 25% trichloroacetic acid, and the reaction mixture was centrifuged at 3,000 r.p.m. for 10 minutes. To 0.5 ml of the supernatent was added 0.5 ml of 0.1% nitrous acid, and after 3 minutes 0.05% solution of N-(l-naphthyl)etnylenediamine dihydrochloride in ethanol was added thereto. The mixture was allowed to stand at 37° C for 25 minutes and then the absorbance at 570 nm was measured. The enzymatic reaction at each concentration was estimated by the following equation and the 50% inhibition concentration ($IC_{50}$ value) was determined from the activity values obtained.

Enzyme Activity Unit = ΔOD x 0.42 x Dilution ratio (µmol/min/ml)

Examples of such measurements are shown in Table 1 below.

Table 1

| | $IC_{50}$ value |
|---|---|
| Compound of Example 1 | 10 µM |
| Compound of Example 2 | 250 µM |

The anti-amnestic effect of the thioproline derivatives of the general formula (I) mentioned above was demonstrated by the experimental method as follows.

Experimental method

A step-down passive avoidance test on rats was used as the method of assay for anti-amnestic activity to estimate the preventive effect of prolylendopeptidase inhibitors on scopolamine-induced amnesia. This method of assay involved application of electric shocks to animals upon their stepping down on a grid floor to see whether they have learned that such shocks can be avoided by staying on a platform. A passive avoidance test chamber used in this experiment was comprised of an electrifiable grid floor (20 cm in length and 22 cm in width) and a platform (20 cm in length, 15 cm in width and 5 cm in height) placed at the right back corner thereof.

5-Weeks-old male rats of Wister strain weighing 100-150 g (purchased from Japan Clea Laboratory) were used in the test.

Each test compound used in the doses indicated in Tables 1 - 4 was dissolved or suspended in 4% gum arabic-containing distilled water (1.0 ml) and administered p.o. one hour before the commencement of acquisition trial. In addition, scopolamine hydrobromide dissolved in saline (0.5 ml) was intraperitoneally administered in a dose of 0.5 mg/kg 30 minutes before the acquisition trial to prepare experimental animal models of amnesia.

In the acquisition trial, the rat was placed on the platform in the test chamber and, when the animal stepped down on the grid floor, the electric current of 5.0 mA was immediately sent to the grid floor continuously until the animal went up on the platform. If the rat having gone up on the platform stayed there for more than 30 seconds, the animal was regarded to have learned the situation and taken out of the test chamber. A total time of measurement in the acquisition trial was limited to 300 seconds as maximum, and the time elapsing until the rat learned the situation was measured. In order to avoid any significant difference in the rats, those which took more than 300 seconds for the learning were excluded from the experiment.

The retention trial was carried out 24 hours after the acquisition trial. In this trial, the rat was placed again on the platform in the test chamber, the time elapsing until the animal stepped down onto the grid floor (the step-down latency) was measured.

The results are shown in Tables 2 and 3. In each of these tables they are indicated as relative step-down latency, shown by each test compound-treated group in the retention trial, to step-down latency for scopolamine-treated group (i.e. test compound-treated group's step-down latency scopolamine-treated group's step-down latency).

Abbreviations used to indicate test compounds in Table 2 are as follows:

ZTT : Benzyloxycarbonyl-L-thioprolyl-L-thioprolinal (control)

ZTTM : Benzyloxycarbonyl-L-thioprolyl-L-thioprolinal dimethyl acetal (compound of Example 1)

ZTTE : Benzyloxycarbonyl-L-thioprolyl-L-thioprolinal diethyl acetal (compound of Example 2)

Table 2

Anti-amnestic effect of the test compounds by oral administration in retention trial

| Test compound | Dose (mg/kg, p.o.) | Scopolamine treatment (mg/kg, i.p.) | Relative step-down latency in retention trial (mean±S.E.) |
|---|---|---|---|
| Control | - | - | 6.5±1.5 |
| Scopolamine | - | 0.5 | 1.0±0.3 |
| ZTT | 0.008 | 0.5 | 1.0±0.3 |
| ZTT | 0.04 | 0.5 | 3.0±1.2** |
| ZTT | 0.2 | 0.5 | 2.6±0.9** |
| ZTT | 1 | 0.5 | 1.6±0.8 |
| ZTT | 5 | 0.5 | 1.0±0.2 |
| ZTT | 25 | 0.5 | 1.4±0.9 |
| Control | - | - | 7.1±1.7 |
| Scopolamine | - | 0.5 | 1.0±0.3 |
| ZTTM | 0.008 | 0.5 | 0.9±0.1 |
| ZTTM | 0.04 | 0.5 | 2.4±1.1 |
| ZTTM | 0.2 | 0.5 | 2.5±1.5 |
| ZTTM | 1 | 0.5 | 1.4±0.3 |
| ZTTM | 5 | 0.5 | 1.1±0.4 |
| ZTTM | 25 | 0.5 | 1.6±0.6 |
| Control | - | - | 7.2±1.6 |
| Scopolamine | - | 0.5 | 1.0±0.3 |
| ZTTE | 0.008 | 0.5 | 1.5±0.6 |
| ZTTE | 0.04 | 0.5 | 2.7±1.4 |
| ZTTE | 0.2 | 0.5 | 1.7±0.9 |
| ZTTE | 1 | 0.5 | 2.3±1.2 |
| ZTTE | 5 | 0.5 | 1.5±0.6 |
| ZTTE | 25 | 0.5 | 1.3±0.7 |

Significantly different from scopolamine-treated group,

** $p<0.01$ (Mann-Whitney's U-test)

Table 3

Anti-amnestic effect of aniracetam by oral administration in retention trial

| Test compound | Dose (mg/kg, p.o.) | Scopolamine treatment (mg/kg, i.p.) | Relative step-down latency in retention trial (mean±S.E.) |
|---|---|---|---|
| Control | - | - | 4.9±0.7 |
| Scopolamine | - | 0.5 | 1.0±0.3 |
| aniracetam | 12.5 | 0.5 | 1.1±0.4 |
| aniracetam | 25 | 0.5 | 1.6±0.6 |
| aniracetam | 50 | 0.5 | 2.3±0.6* |
| aniracetam | 100 | 0.5 | 0.7±0.3 |

Significantly different from scopolamine-treated group,

* $p<0.05$ (Mann-Whitney's U-test)

From these and other test results, it has become evident that the compounds of the general formula (I) mentioned above possess an excellent anti-amnestic action and are thus widely utilizable as low-toxicity medicaments for the remedy of memory disorders such as senile dementia of Alzheimer's type.

The administration of N-benzyloxycarbonyl-glycyl-L-prolyl-chloromethane and N-benzyloxycarbonyl-glycyl-L-prolyl-diazomethane may be effected in various ways, for example by injection such as intravenous, subcutaneous or intramuscular injection or by the oral route. Oral or intravenous administration is particularly preferred. The daily dose of these compounds is preferably between 1 mg and 900 mg, particularly between 5 mg and 500 mg in the case of oral administration, and between 0.5 mg and 500 mg, particularly between 1 mg and 200 mg in the case of intravenous administration.

The preparation of anti-amnestic dosage forms in accordance with the present invention may be effected by any method conventionally used for preparing different pharmaceutical preparations, the choice of appropriate such method being dependent upon the type of the desired dosage form. Examples of such preparations include forms appropriate for oily substances to be absorbed through the gastrointestinal tract, preferably soft capsules and oral liquid preparations.

Soft capsules for oral administration are a unit dosage form and may contain solubilizers such as macrogol and propylene glycol, preservatives such as ethyl p-hydroxybenzoate, propyl p-hydroxybenzoate and sorbic acid, and optionally flavoring agents, dyestuffs, aromatics and the like.

Liquid preparations for oral administration include aqueous or oily suspensions, solutions, syrups, elixirs and the like. Such liquid preparations may contain any additives customary in the art, for example, suspending agents such as sorbit syrup, methyl cellulose, gelatin, hydroxyethyl cellulose, carboxymethyl cellulose, aluminum stearate gel or hydrogenated edible fats; emulsifying agents such as lecithin, sorbitan monooleate and gum arabic (acacia); non-aqueous vehicles such as almond oil, fractionated coconut oil, oily esters, propylene glycol and ethyl alcohol; antiseptics such as methyl p-hydroxybenzoate, propyl p-hydroxybenzoate and sorbic acid; and optionally dyestuffs, aromatics and the like.

Preparations for injection may be in form of ampoules with unit doses, or contained, optionally together with such additives as antiseptics and solubilizers, in a multiple-dose container. Such preparations may take any dosage form, such as suspension, solution and emulsion in oily or aqueous vehicles, and contain such additives as suspending agents.

In the anti-amnestic agents of the present invention, the compounds of the general formula (I) mentioned above as active ingredient will generally be contained in concentrations of not less than 0.1%, preferably 1 - 50% by weight, depending upon the dosage form used.

The following examples illustrate the preparation of the anti-amnestic agents of the present invention, although it is not limited to these examples.

Example of Pharmaceutical Preparation 1

Injection preparations

| | |
|---|---|
| N-Benzyloxycarbonyl-glycyl-L-prolyl-chloromethane | 10 mg |
| Hardened castor oil polyoxyethylene 60 mol ether | 40 mg |
| Sorbitan monostearate | 2 mg |
| Propylene glycol | 60 mg |
| Refined soybean lecithin | 2 mg |
| Cholesterol | 1 mg |
| Glucose | 50 mg |
| Distilled water | to make 1 ml |

Preparation

N-Benzyloxycarbonyl-glycyl-L-prolyl-chloromethane, hardened castor oil polyoxyethylene 60 mol ether, sorbitan monostearate, propylene glycol, refined soybean lecithin and cholesterol are mixed and fused to form a homogeneous liquid in a water bath heated at about 80°C. To this liquid is added with stirring distilled water heated at about 80°C to form a solubilized homogeneous system. Glucose is then added and distilled water is added to make the volume of 1 ml. The liquid is subjected to sterilizing filtration, and charged into an amber ampoule which is then sealed.

Example of Pharmaceutical Preparation 2

Soft capsulated preparations

| | |
|---|---|
| N-Benzyloxycarbonyl-glycyl-L-prolyl-diazomethane | 20 mg |
| Macrogol 400 | 350 mg |
| Propylene glycol | 38 mg |
| Dipotassium glycyrrhizinate | 1 mg |
| Menthol oil | 1 mg |
| Gelatin | 122 mg |
| Glycerol | 30.5 mg |
| D-Sorbitol liquid | 12.2 mg |
| Ethyl p-hydroxybenzoate | 0.8 mg |
| Propyl p-hydroxybenzoate | 0.5 mg |

Preparation

N-Benzyloxycarbonyl-glycyl-L-prolyl-diazomethane, Macrogol 400, dipotassium glycyrrhizinate, menthol oil and propylene glycol are homogeneously blended to form a suspension. Separately, a coating agent for soft capsules is manufactured from gelatin, glycerol, D-sorbitol liquid, ethyl p-hydroxybenzoate and propyl p-hydroxybenzoate. Using the suspension and the coating agent, a soft capsule is prepared.

## Claims

1. New thioproline derivatives of the general formula

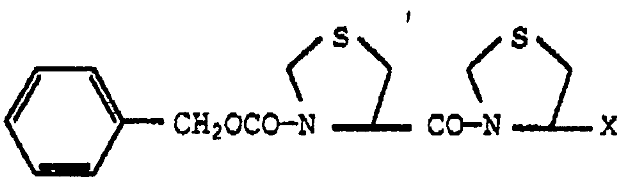

(I)

wherein X is a dialkyl acetal group, a dialkyl thioacetal group, a cyclic acetal group, a cyclic dithioacetal group, a cyclic thioaminal group or a diacyloxymethyl group; a hydroxyiminomethyl group, a lower-alkylaminoiminomethyl group, an arylaminoiminomethyl group or a carbamoylaminoiminomethyl group; or a 2-benzimidazole group.

2. Benzyloxycarbonyl-L-thioprolyl-L-thioprolinal dimethyl acetal.

3. Benzyloxycarbonyl-L-thioprolyl-L-thioprolinal diethyl acetal.

4. Benzyloxycarbonyl-L-thioprolyl-L-thioprolinal dibutyl acetal.

5. Benzyloxycarbonyl-L-thioprolyl-L-thioprolinal dimethylenedioxy acetal.

6. Benzyloxycarbonyl-L-thioprolyl-L-thioprolinal trimethylenedioxy acetal.

7. Benzyloxycarbonyl-L-thioprolyl-L-thioprolinal dimethylene dithioacetal.

8. Benzyloxycarbonyl-L-thioprolyl-L-thioprolinal trimethylene dithioacetal.

9. Benzyloxycarbonyl-L-thioprolyl-L-thioprolinal dimethylene thioaminal.

10. Benzyloxycarbonyl-L-thioprolyl-L-thioprolinal aldoxime.

11. Benzyloxycarbonyl-L-thioprolyl-L-thioprolinal semicarbazone.

12. Benzyloxycarbonyl-L-thioprolyl-L-thioprolinal phenylhydrazone.

13. Benzyloxycarbonyl-L-thioprolyl-L-thioprolinal diacetyl acetal.

14. N-(Benzyloxycarbonylthioprolyl)-4-(2-benzimidazoyl)-thiazolidine.

15. An anti-amnestic agent which comprises at least one thioproline derivative of the general formula (I) shown above as active ingredient.

16. An anti-amnestic agent according claim 15, wherein said thioproline derivative is selected from the compounds according to claims 2 - 14.

17. A proline endopeptidase inhibitor which comprises at least one thioproline derivative of the general formula (I) shown above as active ingredient.

18. A proline endopeptidase inhibitor according to claim 17 wherein said thioproline derivative is selected from the compounds according to claims 2 - 14.

# INTERNATIONAL SEARCH REPORT

International Application No PCT/JP89/01256

**I. CLASSIFICATION OF SUBJECT MATTER** (if several classification symbols apply, indicate all) [6]

According to International Patent Classification (IPC) or to both National Classification and IPC

Int. $Cl^5$ C07D277/06, C07D417/14, C12N9/99, A61K31/425

**II. FIELDS SEARCHED**

Minimum Documentation Searched [7]

| Classification System | Classification Symbols |
|---|---|
| IPC | C07D277/06, C07D417/14, C12N9/99, A61K31/425 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Chemical Abstracts, Abstract NO.110(3): 20344h (J. Biochem. 104(4), 580-6(1988) Tsuru, Daisuke; Yoshimoto, Tadashi; Koriyama, Nobuhiro; Furukawa, Sunao: Abstract of "Thiazolidine derivatives as potent inhibitors specific for prolyl endopeptidase") | 1 - 18 |
| A | JP, A, 64-6263 (Ono Pharmaceutical Co., Ltd.), 10 January 1989 (10. 01. 89), (Family: none) | 1 - 18 |
| A | JP, A, 64-42475 (Kissei Pharmaceutical Co., Ltd.), 14 February 1989 (14. 02. 89) & EP, A, 303434 & US, A, 4857524 & JP, A, 1-226880 | 1 - 18 |
| A | JP, A, 1-250370 (Zeria Pharmaceutical Co., Ltd.), | 1 - 18 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 5, 1990 (05. 02. 90) | March 19, 1990 (19. 03. 90) |
| **International Searching Authority** | **Signature of Authorized Officer** |
| Japanese Patent Office | |